(19) Europäisches Patentamt
European Patent Office
Office européen des brevets

(11) Veröffentlichungsnummer: **0 022 959**
**B1**

(12) # EUROPÄISCHE PATENTSCHRIFT

(45) Veröffentlichungstag der Patentschrift: **26.05.82**

(51) Int. Cl.³: **C 07 C 113/04,** C 07 F 5/02, C 07 C 17/22, C 07 C 25/13

(21) Anmeldenummer: **80103766.4**

(22) Anmeldetag: **02.07.80**

(54) **Verfahren zur Herstellung von Diazoniumtetrafluoroboraten in verdünnter wässriger Lösung.**

(30) Priorität: **14.07.79 DE 2928486**

(43) Veröffentlichungstag der Anmeldung: **28.01.81 Patentblatt 81/4**

(45) Bekanntmachung des Hinweises auf die Patenterteilung: **26.05.82 Patentblatt 82/21**

(84) Benannte Vertragsstaaten: **BE CH DE FR GB IT LI NL**

(56) Entgegenhaltungen:
**EP-80-103766.4**
**GB-A-833163**
**US-A-2606183**
**JOURNAL OF THE AMERICAN CHEMICAL SOCIETY, Band 58, Nr. 11, 1936, MELVIN F.W. DUNKER et al.: «The preparation of some organic mercurials from diazonium borofluorides», Seiten 2308-2309** →

→ **CHEMICAL ABSTRACTS, Band 55, Nr. 11, 29. Mai 1961, Zusammenfassung Nr. 10373f, Columbus, Ohio, US, J.M.W. SCOTT: «Orton rearrangement in aprotic solvents. Some observations on the reactions of p-substituted N-bromoacylanilides in chlorobenzene and trichloracetic acid»**

(73) Patentinhaber: **BAYER AG, Zentralbereich Patente, Marken und Lizenzen, D-5090 Leverkusen 1, Bayerwerk (DE)**

(72) Erfinder: **Hagemann, Hermann, Dr., Roggendorfstrasse 55, D-5000 Köln 80 (DE)**
Erfinder: **Klauke, Erich, Dr., Eichendorffweg 8, D-5068 Odenthal (DE)**

**EP 0 022 959 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von Diazoniumtetrafluoroboraten in verdünnter wässriger Lösung

Die vorliegende Erfindung betrifft ein Verfahren zur Herstellung von Diazoniumtetrafluoroboraten in verdünnter wässriger Lösung.

3-Brom-4-fluortoluol wird erhalten durch Zersetzung von 2-Brom-4-methyl-phenyldiazoniumtetrafluoroboraten. Es ist bekannt, dass man das 2-Brom-4-methyl-phenyldiazoniumtetrafluoroborat durch Diazotierung des 3-Brom-4-aminotoluols bzw. dessen Hydrochlorids mit Natriumnitrit in wässriger Tetrafluoroborsäurelösung herstellen kann, jedoch in lediglich 73,5%iger Ausbeute. Wegen ungenügender Reinheit des so hergestellten Tetrafluoroborats werden bei der sich anschliessenden Zersetzung nur 43% an 3-Brom-4-fluortoluol erhalten (J.M.W. Scott, Can. J. Chem. 38, 2444 (1960)). Nach diesem Verfahren wird in konzentrierten wässrigen Lösungen gearbeitet, so dass man schon kurz nach Beginn der Reaktion schwer durchmischbare Suspensionen erhält. Die Folgen davon sind schlechte Wärmeabführung, lange Reaktionszeiten und qualitativ schlechte Diazoniumtetrafluoroborate, so dass eine grosstechnische Durchführung der Reaktion schwierig ist.

Aus J. Am. Chem. Soc., Vol. 58 (1936), S. 2308-2309 ist die Umsetzung substituierter aromatischer Amine mit wässrigen Tetrafluoroboratlösungen bekannt. Es wird dabei jedoch ebenfalls in konzentrierten Lösungen gearbeitet, wobei die Diazoniumtetrafluoroborate in unbefriedigender Reinheit anfallen.

Dasselbe trifft zu für die aus US-P-2606183 und GB-Pat. 839163 (Beispiel 5A) bekannte Reaktion zur Herstellung der Diazoniumtetrafluoroborate.

Es wurde nun ein Verfahren zur Herstellung von Diazoniumtetrafluoroboraten der Formel (I) gefunden,

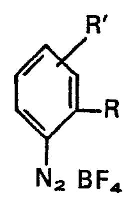

I

in welcher:
R und R' für Alkyl, Halogen, $SCF_3$, $OCF_3$ stehen, das dadurch gekennzeichnet ist, dass man Aniline der Formel (II) oder deren Säureadditionssalze,

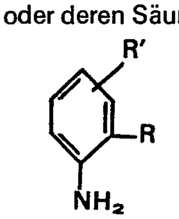

II

in welcher:
R und R' die oben angegebene Bedeutung besitzen, mit Natriumnitrit in wässriger Tetrafluoroborsäure umsetzt, wobei ein 30 bis 200%iger Überschuss an Tetrafluorborsäure im Verhältnis zum eingesetzen Anilin in stark verdünnter wässriger Lösung angewendet wird.

Überraschenderweise erhält man die Diazoniumtetrafluoroborate in sehr guten Ausbeuten (bis zu 95%) und ausgezeichneter Reinheit, wie durch die nachfolgend durchgeführten Zersetzungsreaktionen gezeigt wird.

Verwendet man das 3-Brom-4-aminotoluol als Ausgangsmaterial, so kann der Reaktionsverlauf durch die folgende Reaktionsgleichung wiedergegebenen werden:

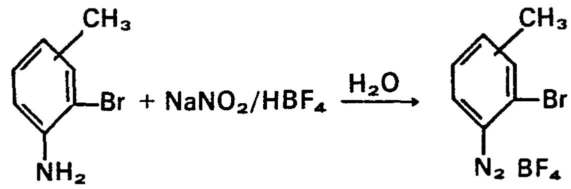


Als Ausgangsverbindungen werden bevorzugt Aniline der Formel (II) oder deren Säureadditionsverbindungen eingesetzt, bei denen R und/oder R' Methyl, Ethyl, Brom, Chlor, Fluor, $SCF_3$ und $OCF_3$ bedeuten.

Die erfindungsgemäss verwendbaren Aniline sind bekannt. Als Beispiele seien 3-Brom-4-aminotoluol und 6-Chlor-2-aminotoluol genannt.

Die Durchführung der Reaktion erfolgt in wässrigem Medium bei Temperaturen zwischen −20° und +30°C, vorzugsweise bei −10° bis +5°C.

Bei der Durchführung des erfindungsgemässen Verfahrens setzt man auf 1 Mol Anilin 1,3 bis 3 Mol Tetrafluorborsäure, vorzugsweise 1,5 bis 2,0 Mol, ein. Die Diazotierung erfolgt mit 20 bis 60%igen wässrigen Alkalinitrit-Lösungen, wobei zweckmässig 1,05 bis 1,1 Mol Nitritsalz auf 1 Mol Anilin verwendet werden.

Es kann eine technisch hergestellte 5 bis 60%iger wässrige Tetrafluorborsäure-Lösung eingesetzt werden. Bevorzugt wird eine 10 bis 40%ige Tetrafluorborsäure-Lösung verwendet, ganz besonders bevorzugt ist eine 15 bis 25 %ige Lösung.

Die als Feststoffe anfallenden Diazoniumtetrafluoroborate werden abfiltriert. Zur Entfernung des anhaftenden Wassers wäscht man mit polaren organischen Lösungsmitteln (vorzugsweise Alkoholen), dann mit einem unpolaren organischen Lösungsmittel und trocknet anschliessend im Vakuum oder bei Normaldruck bei Temperaturen bis zu 50°C.

Die so gewonnenen Diazoniumsalze werden anschliessend der thermischen Zersetzung unterworfen, die durch folgendes Reaktionsschema wiedergegeben wird, R und R' haben dabei die oben angegebene Bedeutung:

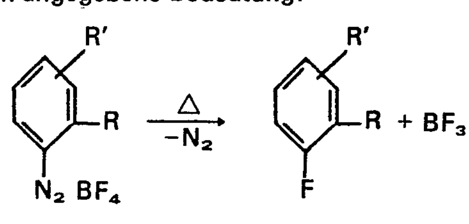

Zur Durchführung der Reaktion können die Diazoniumtetrafluoroborate in Substanz bis zu ihrer Zersetzungstemperatur erhitzt werden. Zur Verfahrensdurchführung (wegen besserer Dosierbarkeit und Steuerung der Temperatur der Zersetzungsmasse) ist es jedoch wesentlich günstiger, die Zersetzung in Gegenwart eines geeigneten Verdünnungsmittels durchzuführen. Beschrieben ist, dass die Zersetzung in Gegenwart von Sand durchführbar ist, jedoch schliesst sich eine aufwendige Abtrennung des Reaktionsproduktes an (s. z.B. J.M.W. Scott, Can. J. Chem. 38 (1960), 2444). Es wurde nunmehr gefunden, dass die Zersetzung auch in inerten organischen Lösungsmitteln, am vorteilhaftesten jedoch in dem erwünschten Reaktionsprodukt selbst als Verdünnungsmittel, vorgenommen werden kann. Dazu werden die festen Diazoniumtetrafluoroborate mit Hilfe geeigneter Einrichtungen (z.B. einer Schnecke) oder als Suspension, beispielsweise mit einer Dosierpumpe, kontinuierlich in das auf die jeweilige Diazoniumsalz-Zersetzungstemperatur gebrachte Reaktionsgefäss, in dem der als Reaktionsprodukt zu synthetisierende kernfluorierte Aromat als Verdünnungsmittel vorgelegt ist, gebracht. Das molare Verhältnis von zu zersetzendem Diazoniumtetrafluoroborat zu Verdünnungsmittel beträgt 1 zu 0,2 bis 2, am vorteilhaftesten 1 zu 0,4 bis 0,8. Aus dem Zersetzungsrückstand wird das gewünschte Produkt mit Hilfe bekannter Methoden (Destillation, Kristallisation) abgetrennt. Die Ausbeuten betragen bis zu 95%.

Das bei der Zersetzungsreaktion anfallende Bortrifluorid wird zweckmässigerweise zur Synthese von Tetrafluoroboraten verwendet. Beispielsweise kann es zur Synthese von Nitrosyltetrafluoroborat mit äquimolaren Mengen von Nitrosylchlorid mit Fluorwasserstoffsäure in Schwefeldioxid oder inerten organischen Lösungsmitteln (z.B. Acetonitril) verwendet werden.

Die erhaltenen kernfluorierten Aromaten sind wertvolle Zwischenprodukte zur Herstellung insektizider Wirkstoffe.

*Beispiele:*

2-Brom-4-methylphenyldiazoniumtetrafluoroborat

In 700 ml 25%iger wässriger Tetrafluorborsäure (2,28 Mol) werden 186 g (0,5 Mol) 3-Brom-4-aminotoluol vorgelegt. Dazu tropft man unter Rühren bei −5° bis 0°C eine Lösung von 76 g (1,1 Mol) Natriumnitrit in 150 ml Wasser. Anschliessend wird das ausgefallene Diazoniumtetrafluoroborat abgesaugt, mit Ethanol und Petrolether gewaschen und getrocknet. Die Ausbeute beträgt 285 g (90%).

Zersetzung des 2-Brom-4-methylphenyldiazoniumtetrafluoroborats zum 3-Brom-4-fluortoluol und Aufarbeitung des Bortrifluorids.

285 g (1 Mol) 2-Brom-4-methylphenyldiazoniumtetrafluoroborat werden mit Hilfe einer Schnecke während 90 min in ein Reaktionsgefäss dosiert, in dem 100 ml 3-Brom-4-fluortoluol auf 100° bis 110°C erwärmt wurden. Das abgespaltene Gasgemisch wird über eine Kolonne in einen unter −10°C gekühlten Stahlautoklaven eingeleitet, der mit 300 ml Schwefeldioxid, in dem 20 g HF und 65 g NOCl gelöst sind, beschickt ist.

Nach beendeter Zersetzung wird das Schwefeldioxid abdestilliert, der kristalline Rückstand kurz im Wasserstrahlvakuum getrocknet. Das ausgefallene Nitrosyltetrafluoroborat (102 g, 87% Ausbeute) kann ohne weitere Reinigung beispielsweise zur Synthese von Diazoniumtetrafluoroboraten eingesetzt werden.

Der angefallene Zersetzungsrückstand wird im Wasserstrahlvakuum destilliert und liefert 170 g (90%) an 3-Brom-4-fluortoluol vom Kp. 72-3°C/18 Torr.

**Patentansprüche**

1. Verfahren zur Herstellung von Diazoniumtetrafluoroboraten der Formel (I),

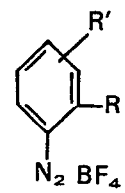

I

in welcher:
R und R′ unabhängig voneinander für Alkyl, Halogen, $SCF_3$, $OCF_3$, stehen, indem man Aniline der Formel (II) oder deren Säureadditionssalze

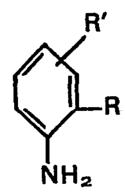

II

in welcher:
R und R′ die oben angegebene Bedeutung besitzen, mit Natriumnitrit in wässriger Tetrafluorborsäure umsetzt, das dadurch gekennzeichnet ist, dass ein 30 bis 200%iger Überschuss an Tetrafluoroborsäure pro Mol eingesetztes Anilin in stark verdünnter wässriger Lösung angewendet wird.

2. Verwendung der nach dem Verfahren gemäss Anspruch 1 erhaltenen Tetrafluoroborate zur Herstellung der entsprechenden Fluorverbindungen, dadurch gekennzeichnet, dass man die Tetrafluoroborate erhitzt.

3. Verwendung der nach dem Verfahren gemäss Anspruch 1 erhaltenen Tetrafluoroborate gemäss Anspruch 2, dadurch gekennzeichnet, dass die Tetrafluoroborate in Anwesenheit der entsprechenden Fluorverbindungen als Verdünnungsmittel erhitzt werden.

4. Verfahren gemäss Anspruch 1, dadurch gekennzeichnet, dass Natriumnitrit in Form seiner 20-60%igen wässrigen Lösung und Tetrafluorborsäure in Form ihrer 5-60%igen wässrigen Lösung eingesetzt wird.

## Claims

1. Process for the preparation of diazonium tetrafluoborates of the formula (I)

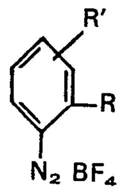

I

in which:
R and R′, independently of one another, represent alkyl, halogen, $SCF_3$ or $OCF_3$, by reacting anilines of the formula (II), or acid addition salts thereof,

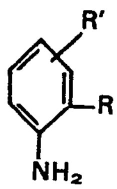

II

in which:
R and R′ have the meaning indicated above, with sodium nitrite in aqueous tetrafluoboric acid, which is characterised in that a 30 to 200% excess of tetrafluoboric acid per mol of aniline employed is used in a very dilute aqueous solution.

2. Use of the tetrafluoborates obtained by the process according to claim 1, for the preparation of the corresponding fluorine compounds, characterised in that the tetrafluoborates are heated.

3. Use, according to claim 2, of the tetrafluoborates obtained by the process according to claim 1, characterised in that the tetrafluoborates are heated in the presence of the corresponding fluorine compounds as diluents.

4. Process according to claim 1, characterised in that sodium nitrite is used in the form of a 20-60% strength aqueous solution and tetrafluoboric acid is used in the form of a 5-60% strength aqueous solution.

## Revendications

1. Procédé de préparation de tétrafluoroborates de diazonium de formule (I):

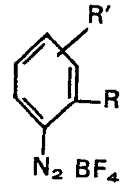

I

dans laquelle
R et R′ représentent indépendamment l'un de l'autre, un groupe alkyle, un atome d'halogène, un groupe $SCF_3$ ou $OCF_3$, en faisant réagir des anilines de formule (II) ou leurs sels d'addition d'acide:

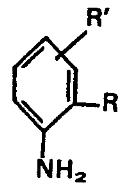

II

dans laquelle
R et R′ ont les significations indiquées ci-dessus, avec du nitrite de sodium dans de l'acide tétrafluoroborique aqueux, caractérisé en ce qu'on utilise un excès de 30 à 200% d'acide tétrafluoroborique par mole d'aniline utilisée dans une solution aqueuse fortement diluée.

2. Utilisation des tétrafluoroborates obtenus conformément au procédé suivant la revendication 1 pour la préparation des composés fluorés correspondants, caractérisée en ce qu'on chauffe les tétrafluoroborates.

3. Utilisation des tétrafluoroborates suivant la revendication 2, obtenus conformément au procédé suivant la revendication 1, caractérisée en ce qu'on chauffe les tétrafluoroborates en présence des composés fluorés correspondants comme agents diluants.

4. Procédé suivant la revendication 1, caractérisé en ce qu'on utilise du nitrite de sodium sous forme d'une solution aqueuse à 20-60% et de l'acide tétrafluoroborique sous forme d'une solution aqueuse à 5-60%.